# EUROPEAN PATENT SPECIFICATION

(11) **EP 1 800 685 B1**
(45) Date of publication and mention of the grant of the patent: **22.08.2012**
(21) Application number: 05791870.8
(22) Date of filing: 29.09.2005
(51) Int. Cl.: A61K 31/7048, A61P 9/10, A61P 9/06, A61P 3/06

(54) **STEROIDAL SAPONIN PHARMACEUTICAL COMPOSITION, ITS PREPARATION METHOD AND USE**
PHARMAZEUTISCHE STEROIDE SAPONIN-ZUSAMMENSETZUNG, IHR HERSTELLUNGSVERFAHREN UND IHRE VERWENDUNG
COMPOSITION PHARMACEUTIQUE A BASE DE SAPONINES STEROIDIQUES, SON PROCEDE DE PREPARATION ET SON UTILISATION

(30) Priority: 30.09.2004 CN 200410040771
(43) Date of publication of application: 27.06.2007
(73) Proprietor: Chengdu Di'ao Pharmaceutical Group Co., Ltd., Chengdu Sichuan 610041 (CN)
(72) Inventor: LIU, Zhongrong, Chengdu,Sichuan 610041 (CN); QI, Wei, Chengdu,Sichuan 610041 (CN); FU, Tiejun, Chengdu,Sichuan 610041 (CN); ZOU, Wenjun, Chengdu,Sichuan 610041 (CN); JI, Yuanqiao, Chengdu,Sichuan 610041 (CN); LI, Bogang, Chengdu,Sichuan 610041 (CN); HUANG, Yu, Chengdu,Sichuan 610041 (CN)
(74) Representative: Le Coupanec, Pascale A.M.P.
(86) International application number: PCT/CN2005/001621
(87) International publication number: WO 2006/034655

(56) References cited:
- CN-A- 1 389 469
- CN-A- 1 415 625
- CN-A- 1 511 535
- KR-A- 2004 077 648
- KWON CHONG-SUK ET AL: "Anti-obesity effect of Dioscorea nipponica Makino with lipase-inhibitory activity in rodents." BIOSCIENCE, BIOTECHNOLOGY, AND BIOCHEMISTRY JUL 2003, vol. 67, no. 7, July 2003 (2003-07), pages 1451-1456, XP002535617 ISSN: 0916-8451
- DONG M ET AL: "Two novel furostanol saponins from the rhizomes of Dioscorea panthaica prain et burkill and their cytotoxic activity" TETRAHEDRON 20010114 GB, vol. 57, no. 3, 14 January 2001 (2001-01-14), pages 501-506, XP002535618 ISSN: 0040-4020
- LI B. ET AL: 'The chemistry of new drug Di'ao Xinxuekang for treatment heart-blod disease' NEW DRUGS AND CLINICAL REMEDIES vol. 13, no. 2, 1994, pages 75 - 76
- 'Chinese Pharmacopoeia 2000th Part 1', CHEMICAL INDUSTRY PUBLISHING COMPANY pages 441 - 442

## Description

### Technical Field of the Invention

The present invention relates to a pharmaceutical composition containing steroidal saponins, particularly, relates to a pharmaceutical composition containing steroidal saponins mainly extracted from the raw materials of *Dioscorea panthaica* Prain et Burkill and *Dioscorea nipponica* Makino.

### Background of the Invention

Steroidal saponins are a sort of important bioactive substance in plants. The study of steroidal saponin has been occupying an important position in chemistry of natural products. The aglycon of steroidal saponin is spirostanol or furostanol containing 27 carbon atoms, and exists mostly in such monocotyledonous plants as Liliaceae, Amaryllidaceae and Dioscoreaceae.

The herb *Dioscorea panthaica* Pain et Burkill (a species in Dioscorea, Dioscoreaceae) is the rootstocks of herbal plant *Dioscorea panthaica* Prain et Burkill and has many other names such as curcuma, curcumae longae, tendrilleaf solomonseal rhizome and Turmeric Root Tuber. It is distributed in Southwest China and Hubei and Hunan provinces of the country and has the actions of regulating *qi* (*vital energy*) to alleviate pain and detumescence by detoxification, so it is often prescribed for peratodynia, vomiting, diarrhea, abdominal pains, injuries, fractures, pyocutaneous diseases and venomous snake bites [Chinese Materia Medica, Vol 8 p8.247, compiled by Chinese Materia Medica Editorial Committee, State Traditional Chinese Medical Administration Bureau, and published by Shanghai Science and Technology Press in 1999]. The herb *Dioscorea nipponica* Makino (a species in Dioscorea, Dioscoreaceae) is the rootstocks of the herbal plant *Dioscorea nipponica* Makino and also owns many other names such as curcuma, monkshood vine root-bark and so on. It is distributed in North, Northeast and Northwest of China and also in Henan, Hubei, Shandong, Jiangsu, Anhui, Zhejiang, Jiangxi and Sichuan provinces of the country. It has the actions of expelling rheumatism and removing wetness, promoting blood circulation to remove meridian obstruction and stopping coughing, so it is often prescribed for rheumatic arthralgia, limb numbness, thoracic obstruction, cardiodynia, chronic tracheitis, injuries, fractures, malaria, anthracia and swelling [Chinese Materia Medica, Vol 8 p238, compiled by Chinese Materia Medica Editorial Committee, State Traditional Chinese Medical Administration Bureau, and published by Shanghai Science and Technology Press in 1999]. *Dioscorea nipponica* Makino was also listed on P435-436, Part I, Edition 1977 of Pharmacopoeia of the People's Republic of China.

Presently, researches on *Dioscorea panthaica* Prain et Burkill and *Dioscorea nipponica* Makino are rare. It has been reported by Bogang Li *et al.* that two kinds of water-insoluble steroidal saponins, dioscin and gracillin, were separated and identified from *Dioscorea panthaica* Prain et Burkill. [Bogang Li et al. ACTA BOTANICA SINICA 1986, 28(4): 409-411*];* Three kinds of steriodal saponins were separated by Mei Dong et al. from the *Dioscorea panthaica* Prain et Burkill, the structures of which have been identified as pseudoprosteroidal saponins (Dioscin 1) and DI-9 (Dioscin 6) *[*Mei Dong et al. ACTA MEDICA SINICA 2001, 36(1): 42-45]. It is also reported that two kinds of water-insoluble steroidal saponins, dioscin and gracillin both being the components of perhexiline, were obtained and identified by Yiwei Fang et al. from *Dioscorea nipponica* Makino [Yiwei Fang et al. ACTA MEDICA SINICA 1982, 17(5):388-391*];* Two kinds of steroidal saponins were also extracted and obtained from *Dioscorea nipponica* Makino, in which one is water-insoluble and another is water-soluble, the structures of which are determined as gracillins [Shuhu Du et al. ACTA MEDICA SINICA 2002, 37(4) : 267-270]. Another research discloses the structural formulas of eight kinds of steroidal saponins extracted from the total dioscins [Bogang Li; Zhengzhi Zhou. Traditional Chinese Drug Research & Clinical Pharmacology 1994, 13(2): 75-76*].* Said eight kinds of steroidal saponins are the active components for treating coronary artery disease, including dioscins, protodioscins, protogracillins and gracillins. Chinese patent application CN020128119.X discloses a new use of six kinds of spirostanol steroidal saponins for treating Cardiovascular Diseases. Chinese patent application CN02112159.1 discloses a new use of a composition containing diosgenins for treating Myocardial Ischemia, Angina and Myocardial Infarction. Until now, only two kinds of components, pseudoprotodioscin (I) and dioscin (II), have been separated and identified from the herbs *Dioscorea panthaica* Prain et Burkill and *Dioscorea nipponica* Makino.

Although the above-mentioned references disclose that *Dioscorea panthaica* Prain et Burkill and *Dioscorea nipponica* Makino contain several kinds of steroidal saponins that are effective for treatment of cardiovascular diseases, the researches only relate to chemical studies on effective components of above two plants, and some of the components have little value in pharmaceuticals due to their low contents in the plants. As everyone knows, it would be very difficult and high cost to obtain effective and pure chemicals from plants. Meanwhile, in order to avoid unexpected side effects and to reduce costs and use conveniently, it is a general practice not to use pure effective compounds of herbal plants but use the extracts of the herbal plants as raw materials of pharmaceuticals. One disadvantage for such practice is that differences of seasons for harvesting the plants and growth areas thereof would result in changes in contents of effective components. However, unclearness of main effective components and the contents thereof would make great difficulties in quality control of pharmaceuticals. Therefore, in order to maintain stability of quality of herbal medicine, it is important to get a clear idea on the basic components and contents of herbal plants. Due to technical limitation and complexity in species in steroidal saponins, there is no definitive report on main effective components of steroidal saponins, and the contents and purity thereof so far.

The traditional method for preparation of total steroidal saponins is as follows: after heat extraction with methanol or ethanol, condensing the alcoholic solution obtained; after removing methanol or ethanol, degreasing the solution with chloroform, extracting with normal butyl alcohol containing water. Total steroidal saponins are achieved after condensing the solution. The process described above has the following shortcomings: 1) low yield and high cost; 2) long production period; 3) in need of great amount of organic solvents, which might be risky of flaming or poisoning in production and bring about serious environmental pollution; 4) great energy consumption resulted from condensation and desiccation of the normal butyl alcohol with a high boiling point; 5) poorness in color and gloss of products and improperness for industrialized production. Total steroidal saponins prepared with traditional techniques are instable in contents, which would cause unstableness in effects of the drugs made thereof.

### Summary of the Invention

In order to overcome the above shortcomings, the present invention provides a pharmaceutical composition containing steroidal saponins. The present invention also provides a method for preparing the same and a use thereof.

The present invention provides a pharmaceutical composition containing steroidal saponins, it comprises furostanol saponin represented by general formula A and saponin or sapogenin represented by general formula C. 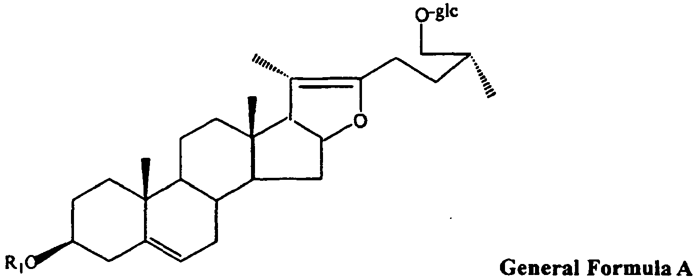 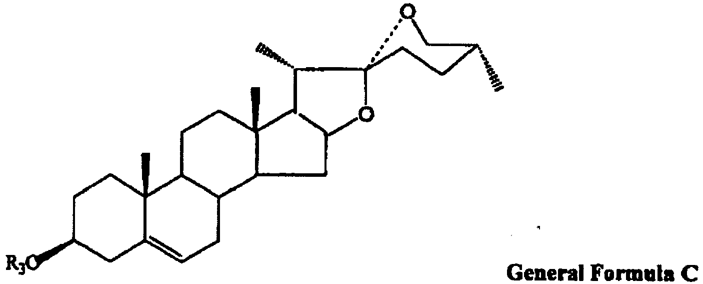 wherein, in general formula A, R₁ = 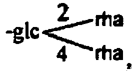 H, 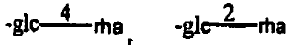 or 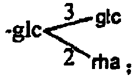 in general formula C: R₃ = 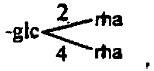 H, -glc,

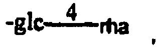

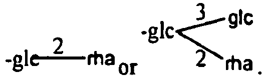

More preferably, it comprises following compounds: in general formula A:
when R₁ = 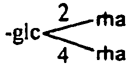 the compound represented by general formula A is pseudoprotodioscin (I);
when R1 = 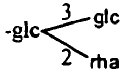 the compound represented by general formula A is pseudoprotogracillin (II);
in general formula B,
when R₂ = 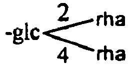
in general formula C, when R₃ = 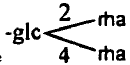
the compound represented by general formula C is dioscin(III),

It comprises 5- to 20 parts of pseudoprotodioscin (I), 1 to 3 parts of pseudoprotogracillin (II) and 1 ~ to 5 parts of dioscin (III).

More preferably, it comprises 12 ~ to 18 parts of pseudoprotodioscin (I), 1 part of pseudoprotogracillin (II) and 1.2 ~ to 2.5 parts of dioscin (III).

The steroidal saponins used in the composition are originated from the extracts of *Dioscorea panthaica* Prain et Burkill and *Dioscorea nipponica* Makino, both belonging to species of Dioscorea, Dioscoreaceae.

Wherein, in the said extract of total steroidal saponins, the content of total steroidal saponins is more than 80% (w/w), and no less than 65% (w/w) when calculated by dioscin.

Wherein, in the said extract, the total content of three kinds of steroidal saponins, i.e. pseudoprotodioscin (I), pseudoprotogracillin (II) and dioscin (III), is no less than 50% (w/w) of the content of total steroidal saponins.

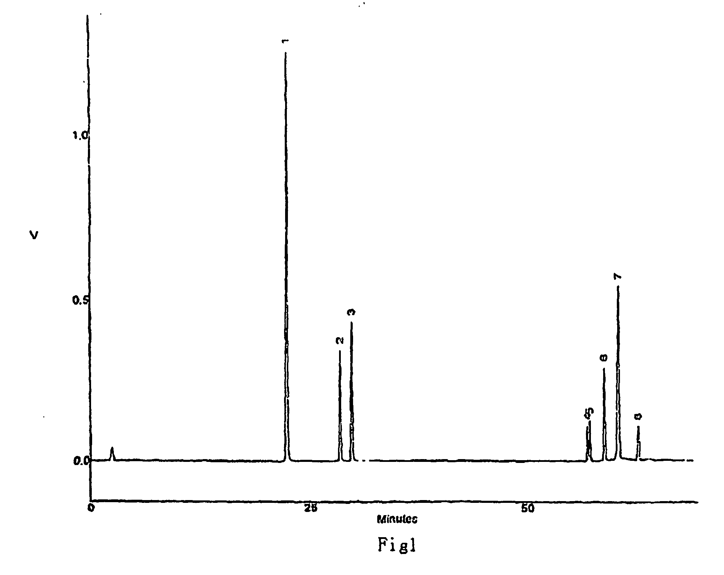

The said extract has the HPLC fingerprint as shown in Fig 1, wherein the characteristic peaks of the fingerprint are as follows respectively: the retention time for pseudoprotodioscin (I) is 28.27 min; the retention time for pseudoprotogracillin (II) is 29.5 min and the retention time for dioscin (III) is 57.10 min.

Chromatographic conditions: chromatographic column: Alltima C18 4.6×250mm, 5µm; Gradient elution; Determined with an evaporative light scattering detector; Drift tube temperature: 100 °C; Gas flow rate: 2.0L / min

The said extract is prepared by the methods as follows:
a. Taking rootstocks of *Dioscorea panthaica* Prain et Burkill and *Dioscorea nipponica* Makino, or the rootstocks of freshly-harvested herbal plants *Dioscorea panthaica* Prain et Burkill and *Dioscorea nipponica* Makino as raw material. Extracting with mixed solution containing water, methanol, ethanol, n-butanol or mixed solution comprising one or more than one kinds of other low-fat alcohols after crushing and slicing the rootstocks, the amount of said mixed solution shall be 15 to 25 times of the raw material.
b. Cooling and filtering the extract prepared in step a, passing the filtrate through absorbent resin column, abandoning the effluent and washing with water till the effluent turns colorless, abandoning the rinsing water.
c. Eluting the absorbent resin column that is washed with water in step b with one or more than one kinds of solvent selected from a group consisting of ethanol, methanol, acetone, 50% to 90 % ethanol, 30 to 80 % hydrous methanol and 60 to 95% hydrous acetone; collecting and condensing the effluent.
d. adding 60 to 95 % alcohol to the concentrated solution obtained in step c for alcohol precipitation, filtering and collecting filtrate, and the product is obtained after condensing and drying the filtrate.

Wherein, in step b of the above method, it further includes a step of decompressing condensation before cooling and filtering when the extract contains methanol, ethanol, n-butanol or other low-fat alcohols.

The solvent used in step c can be one or more than one kinds of solvent selected from water, methanol, ethanol, n-butanol or other low-fat alcohols or mixture thereof. The extraction is conducted by soakage or ultrasonic resonance at room temperature, or by soakage or reflux under heating condition. The times for extraction might be once or several times.

The resins used in the step b can be one or more than one kinds of resins selected from a group consisting of HPD₁₀₀, HPD₃₀₀, LD₄₀, D₁₀₁ and mixture thereof. The solvent for elution can be one or more than one kinds of solvents selected from a group consisting of water, methanol, ethanol, acetone, hydrous methanol, hydrous ethanol and hydrous acetone. Elution can be either concentration elution or gradient elution.

The pharmaceutical composition of the present invention comprises steroidal saponins or the extracts thereof as an active ingredient and pharmaceutically acceptable adjuvants.

Wherein, the pharmaceutical composition of the present invention can be prepared to various forms, such as tablet, capsule, soft capsule, grain, oral liquor, dripping pill and injection.

The present invention also provides a method for preparing the pharmaceutical composition, including the following steps:
Taking by weighing furostanol saponins of general formula A and/or general formula B and spirostanol saponin of general formula C and mixing them at the ratio of 5 to 25 parts by weight of furostanol saponin and 1 to 5 parts by weight of spirostanol saponin. Adding pharmaceutically acceptable adjuvants, it could be then prepared to pharmaceutical composition.

Further, the pharmaceutical composition provided by the present invention can also be prepared by the following method:
a. Taking rootstocks of *Dioscorea panthaica* Prain et Burkill and *Dioscorea nipponica* Makino, or the rootstocks of freshly-collected herbal plants of the same as the raw material. After slicing or smashing the rootstocks, extracting with one or more than one kinds of solvent selected from a group consisting of water, methanol, ethanol, n-butanol and other low-fat alcohols, the amount of the solvent shall be 15 to 25 times of the raw material.
b. Cooling and filtering the extract prepared in step a, passing the filtrate through absorbent resin column, abandoning the effluent and washing with water till the effluent turns colorless, abandoning the rinsing water.
c. Eluting the absorbent resin column that was washed with water in step b with one or more than one kinds of solvent selected from a group consisting of ethanol, methanol, acetone, 50% to 90% ethanol, 30 to 80% hydrous methanol and 60 to 95 % hydrous acetone, collecting and condensing the effluent.
d. Adding 60 to 95 % alcohol to the concentrated solution obtained in step c for alcohol precipitation, filtering and collecting the filtrate.
e. Condensing and drying the filtrate in step d, and adding pharmaceutically acceptable adjuvants to obtain the pharmaceutical composition in the forms of common usage.

Wherein, in step b, it further includes a step of decompressing condensation before cooling and filtering when the extract contains methanol, ethanol, n-butanol or other low-fat alcohols.

The present invention also provides a use of the pharmaceutical composition in preparing the pharmaceutical for treating and preventing cerebrocardiovascular diseases. Wherein, said pharmaceutical refers to that to treat coronary artery disease, angina, myocardial infarction, arrhythmia, hyperlipemia or ischemic cerebrovascular disease.

After determination of the three steroidal saponin components in the pharmaceutical composition of the present invention, it is proved that it has high stability and liability in its therapeutic effect. Meanwhile, the method to identify the three steroidal saponins is provided with reliable controllability. Besides, with its daily dosage of 300 to 600 mg, which is less dosage, the drug of this invention provides a new choice in clinics.

Obviously, according to the above-mentioned content of the present invention and by means of common knowledge and routine measures adopted in this field of technology, it is possible to make many amendments, replacements or alternations to the present invention under the prerequisite not to break away the basic ideas of the techniques mentioned above.

Following embodiments are used to further illustrate the present invention, but this by no means shall be understood as the limitation for the scope of the subjects of the present invention. Any technology realized on basis of the said content of the present invention belongs to the scope of the present invention.

### Brief Description of Drawings

FIG 1 is the HPLC spectrogram for protodioscin sample and the mixture of compounds 1~ to 8, wherein peaks numbered 1 to 8 are correspondent to compounds 1~ to 8;

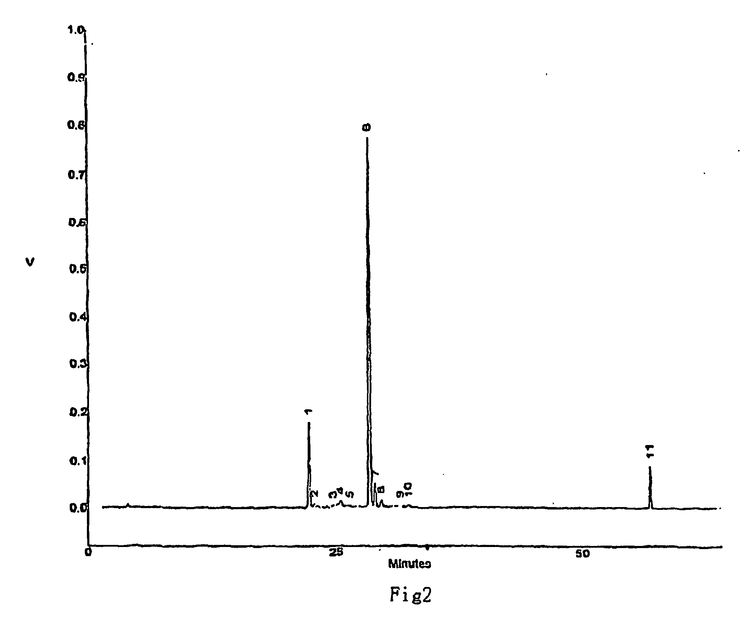

FIG 2 is the HPLC spectrogram for the composition in example 1, wherein the peak No 1 is correspondent to compound 1, the peak No 6 is correspondent to compound 2, the peak No 7 is correspondent to compound 9, the peak No 8 is correspondent to compound 3 and the peak No 11 is correspondent to compound 5.

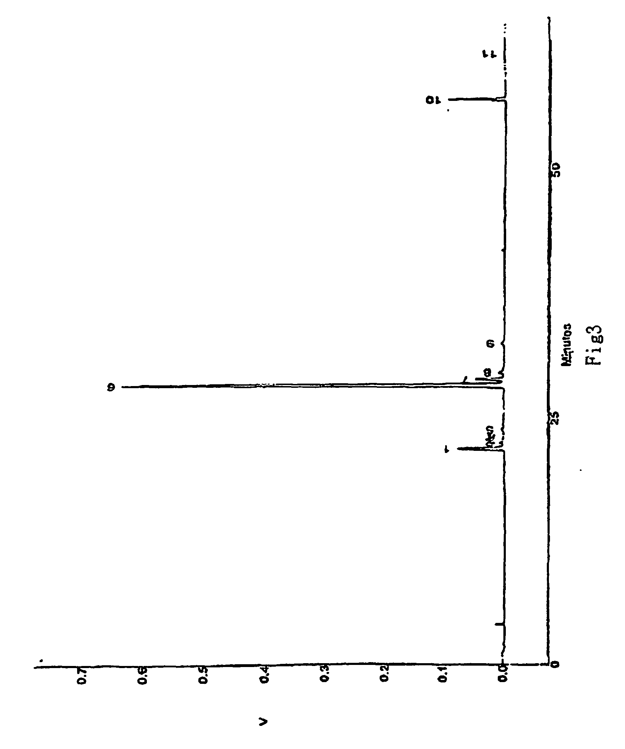

FIG 3 is the HPLC spectrogram for the composition in example 2, wherein the peak No 1 is correspondent to compound 1, the peak No 6 is correspondent to compound 2, the peak No 7 is correspondent to compound 9, the peak No 8 is correspondent to compound 3 and the peak No 10 is correspondent to compound 5.

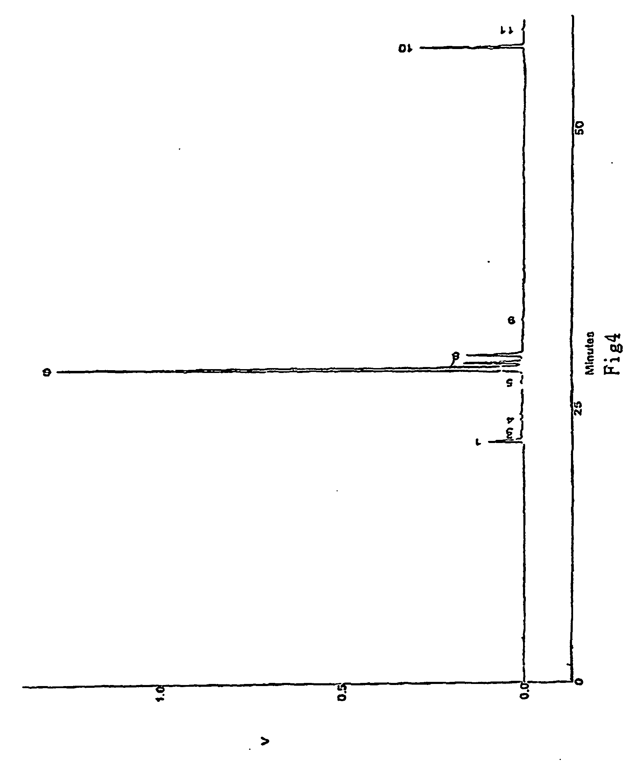

FIG 4 is the HPLC spectrogram for the composition in example 3, wherein the peak No 1 is correspondent to compound 1, the peak No 6 is correspondent to compound 2, the peak No 7 is correspondent to compound 9, the peak No 8 is correspondent to compound 3 and the peak No 10 is correspondent to compound 5.

### Detailed Description of the Invention

### Example 1 Preparation of the pharmaceutical composition of the present invention:

100 kg dried rootstocks of *Dioscorea nipponica* Makino were taken and crushed. Reflux was made with 95% ethanol (1200L×3) for 3 hrs for the first time and then 2 hrs for the second and third times, respectively. After filtration, the extract was gathered and ethanol recovered. With addition of water to 2g / ml, it was refrigerated for 24 hrs before being centrifuged. The supernatant liquid was passed through HPD₃₀₀ resin column of adsorption, which was then washed with water till the effluent turned colorless before being eluted with 70% ethanol. The part eluted with 70% ethanol was collected and condensed. The concentrated solution was added with 75 % ethanol for precipitation before being filtrated. The filtrate was collected, condensed and dried. After being desiccated by vacuum dehydration, the product of total steroidal saponins_of *Dioscorea nipponica* Makino was obtained. The yield was 2.32% and the content of total steroidal saponins was 85 % (w / w).

### Example 2 Preparation of the pharmaceutical composition of the present invention:

100 kg dried rootstocks of herb *Dioscorea panthaica* Prain et Burkill were taken and sliced. They were boiled for 4 times with addition of 12 times of water. The time for first decoction is 3 hrs and then 2 hrs for the second, third and fourth times, respectively. After filtration, the extract was gathered and placed till it turned to room temperature. After being centrifuged, the supernatant liquid was passed through macroporous adsorptive resin column [HPD₁₀₀ / LD₁₀₀=7:3 (W / W)], which was then washed with water till the effluent turned colorless before being eluted with 10 % ethanol. Elution with 75 % ethanol were done finally and the part eluted with 75 % ethanol was collected and condensed. The concentrated solution was added with 80 % ethanol for precipitation before be filtrated. The filtrate was collected, condensed and dried till the filtrate is odorless after ethanol is recovered. After being desiccated again with the method of spray drying, the product of total steroidal saponins of *Dioscorea panthaica* Prain et Burkill was obtained. The yield was 1.5% and the content of total steroidal saponins was 90 % (w/w).

### Example 3 Preparation of the pharmaceutical composition of the present invention:

400 kg dry rootstocks of fresh *Dioscorea nipponica* Makino were taken and sliced. They were boiled for 3 times with addition of 8 times of water. The time for first decoction is 3 hrs and then 2 hrs for the second and third times, respectively. After filtration, the extract was gathered and placed till it cooled down to room temperature. After being centrifuged, the supernatant liquid was passed through macroporous adsorptive resin column [HPD₁₀₀ / LD₁₀₀=6:4(w/w)], which was then flushed and back flushed thoroughly with water before being eluted with 10% ethanol. Elution with 80 % ethanol were done finally and the part eluted with 80 % ethanol was collected and condensed. The concentrated solution was added with 90% ethanol for alcohol precipitation before being filtrated. The filtrate was collected, condensed, and dried till the filtrate is odorless after ethanol is recovered. After being desiccated again with the method of spray drying, the product of total steroidal saponins of *Dioscorea nipponica* Makino was obtained with a yield of 0.56% and the content thereof was 95 % (w/w).

Example 4 Preparation and identification of the compounds in the pharmaceutical composition of the present invention:

1278 g crude extract obtained in example 1 was taken and dispersed in 11100 ml water. Extraction was made in turn with acetic ether (7500mlx4) and n-butanol (7500ml×5), obtaining 102 g extract of acetic ether and 503 g extract of n-butanol. 236 g of the part of n-butanol extract was taken for silica gel column chromatography (I10.5×52cm, 2000g) and then gradiently eluted with chloroform-methanol-water (9 : 1: 0.1). Each flask recovering 500 ml eluent, total 160 parts were collected. Compound 8 (140mg) was separated out with Fr.16-21, and compound 7 (427mg) was separated out with Fr.41-45, and compound 6 (30g) with Fr. 56-65, compound 5 (8.62 g) with Fr. 94-104, which is the steroidal saponin (III) of the present invention. Fr.113-127 was combined as E (26g), which was separated with silica gel columns (I6.5×71cm, 800g) and eluted with chloroform-methanol (5:1) saturated with water. With E passing through the column, Fr.14-20 was combined with as B (4 g), and after ODS purification compound 4 (1.3 g) was obtained. Fr.128-143 was merged as F (63 g), which was separated with silica gel columns (II7×52cm, 850 g) and eluted with chloroform-methanol (5:1) saturated with water. With F passing through the column, Fr. 36-60 was combined with as A (10 g) and compound 3 (115 mg) was obtained after ODS purification. With F passing through the column, Fr.61-68 was combined with as B (11 g) and compound 9 (105 mg) was obtained after ODS purification, which was the steroidal saponin (II) of the present invention. With F passing through the column, Fr.69-78 was combined with as C (10 g) and compound 2 (2.89 g) was obtained after ODS purification, which was the steroidal saponin (I) of the present invention.

The silica gel used for column chromatography (160-200 M, 200-300 M) and GF254 silica gel used for TLC are the products of Qingdao Oceanic Chemical Factory. The reverse phase silica gel ODS (Cosmosi175 C₁₈-OPN) is the product of Japan Nacalai Tesque Company and the reverse phase silica gel plate RP-18F₂₅₄ is the product of Merck.

With modern method of spectrum of MS, ¹HNMR, ¹³C NMR, DEPT, HMQC and HMBC and in combination with physical and chemical parameters, the structures of the compounds were identified as follows:
The mass spectrometer used was Finnigan LCQ^{DECA} , and the nuclear magnetic resonance analyzer in use was Bruker AM-400 with TMS as internal standard.

### Compound 9 (pseudoprotogracillin)

¹³C NMR data of compound 9 was compared with those of pseudoprotodioscin^{[3]} and gracillin^{[4]}. The results showed that the sugar signal binding to 26-C of compound 9 and the chemical shift in aglycon part thereof were totally in accord with those of pseudoprodiocin, and the extra-sugar signals accorded completely with those of gracillin. Accordingly, it was judged primarily that compound 9 was the product of protogracillin dehydrating one H₂O molecule at C₂₀ to C₂₂ (namely pseudoprotogracillin (II)). In the spectrum of HMBC, 1-H-glc""(δ4.83) is correlated with aglycon 26-C(δ_{C} 74.7), 1-H-rha"(δ_{H} 6.40) and 1-H-glc"' (δ_{H} 5.10) with 2-C-glc' (δ_{C} 76.8) and 3-C-glc' (δ_{C} 89.3), respectively, and 1-H-glc' (δ_{C} 4.95) with 3-C(δ_{c}, 77.7). All this also proved the above-presumed binding position and sequence of sugar.

In summary, the structure of compound 9 is identified as 26-0-β-D-glucopyranosyl-3β, 26 glyco-25(R)-Δ^{5,20(22)}-diene-furo-3-O-[α-L-Rhamnop yranosyl(1 → 2)]-[β-D-glucopyranosyl(1 → 3)]-β-D-glucopyranoside,namely pseudoprotogracillin (II).

Compound 8 (trillin): White needle crystals. ESIMS *m*/*z* : 577[M+H]⁺, 415[M-glc+H]⁺, 397[M-gle-H₂O+H)⁺, 1151[2M-H]⁻, 575[M-H]⁻; ¹HNMR (400 MHz, C₅D₅N): δ1.11(3H, d, CH₃-21, *J*=6.8 Hz), 0.87(3H, s, CH₃-19), 0.80 (3H, s, CH₃-18), 0.67 (3H, d, CH₃-27, *J*=4.6 Hz) present 4 signals of methyl proton of the aglycon of dioscin. ¹³C NMR(100 MHz, C₅D₅N) is shown in Table 1. The aglycon part was compared with that of dioscin, wherein in C-3, δ71.7→78.6, in C-2, δ31.4→ 30.2, in C-4, δ42.3→39.3, all illustrate that C-3 part of aglycone is bound to sugar. The ¹³C NMR data of compound 8 (as shown in Table 3) is in accordance with those reported in reference 1 and 2.

Compound 7 (Progenin II: 3-0-[α-L-rhamnopyranose (1→4)]-β-D-glucopy ranose-dioscin): White needle crystals. ESI-MS *m*/*z*: 723 [M+H]⁺, 577 [M-rha+H]⁺, 415[M-rha-glc+H]⁺, 397 [M-rha-glu-H₂O+H]⁺, 721 [M-H]⁻, 575 [M-rha-H]⁻; ¹H NMR (400 MHz, C₅D₅N): δ1.11(3H, d, CH₃-21, *J*=6, 9 Hz), 0.88 (3H, s, CH₃-19), 0.80 (3H, s, CH₃-18), 0.66 (3H, d, CH₃-27, *J*=5.2 Hz) present 4 signals of methyl proton of the aglycon of dioscin. The anomeric proton δ4.94 (1H, d, *J*=7.7Hz) of glucose shows that it belongs to β type and the anomeric proton 6.54(1 H, βr,s) of rhamnose shows that it belongs to a type. The ¹³C NMR data of compound 7 (as shown in Table 3) is in accordance with those reported in reference 1 and 2.

Compound 6 (Progenin III: 3-O -[α-L-rhamnopyranose (1→2)]-β-D-glu copyranose-dioscin): White powder. ESI-MS *m*/*z*: 723 [M+H]⁺, 577 [M-rha+H]⁺, 415[M-rha-glc+H]⁺, 397 [M-rha-glc-H₂O+H]⁺; ¹H NMR (400 MHz, C₅D₅N): δ1.11(3H, d, CH2 *J*=7.0 Hz), 1.02 (3H, s, CH₃-19), 0.80 (3H, s, CH₃-18), 0.66 (3H, d, CH₃-27, *J*=5.0 Hz) present 4 signals of methyl proton of the aglycon of dioscin. δ6.36 (1H, br, s) of rhamnose shows that it belongs to a type. 85.04 (1H, d, *J*=9.0 Hz) of glucose shows that it belongs to β type. The ¹³C NMR data of compound 6 (see Table 3) is in accordance with those reported in reference 1 and 2.

Compound 5 (dioscin): White needle crystals. ESI-MS *m*/*z:* 891[M+Na]⁺, 869[M+H]⁺, 577[M-rha-rha+H]⁺, 437[M-rha-rha-gle+Na]⁺, 903[M+Cl]⁻, 867[M-H]⁻, 721[M-rha-H]⁻; ¹H NMR(400 MHz, C₅D₅N): δ1.74 (3H, d, *J*=6.5 Hz), 1.60(3H, d, *J*=6.2 Hz) present 2 signals of methyl proton of rhamnose, respectively. 86.37 (1H, br, s), 5.83 (1H, br, s) shows these two rhamnoses are of a type. δ4.92(1H, d, *J*=6.4 Hz) shows the glucose is of β type. δ1.11 (3H, d, CH₃-21, *J*=6.8 Hz), 1.02(3H, s, CH₃-19), 0.79 (3H, s, CH₃-18), 0.66 (3H, d, CH₃-27, J=4.9 Hz) present 4 signals of methyl proton of the aglycon of dioscin, respectively. The ¹³C NMR data of compound 5 (see Table 4) is in accordance with those reported in reference 1, and is dioscin.

Compound 4 (3b, 26-diol-(25R) Δ^{5,20(22)}- diene-furo-26-*0*-β-D- glucopyranoside): White powder. ESI-MS *m*/*z:* 907 [M+Na]⁺, 885 [M+H]⁺, 723 [M-glc+H]⁺, 577 [M-glc-rha+H]⁺, 437 [M-glc-rha-glc+Na]⁺, 883 [M-H]⁻; ¹H NMR(400MHz, C₅D₅N): δ1.61(3H, s, CH₃-21), 0.99(3H, d, CH₃-27, *J*=6.4 Hz), 0.89(3H,s, CH₃-19), 0.70(3H, s, CH₃-18) present 4 signals of methyl proton of the aglycone of dioscin. δ4.95(1H, d, *J*=7.5 Hz), 4.82(1H, d, *J*=6.1 Hz) show that the glucose in compound 4 is of β type and δ 5.90(1H, br, s) show that the rhamnose of the same is of a type. In the ¹³C NMR data of compound 4, aglycon part was compared with that of compound 7, wherein in C-3, δ71.2 →78.3, demonstrating, besides C-26 is bound to sugar, C-3 is also bound to sugar. The ¹³C NMR data of compound 4 is in accordance with those reported in reference 4.

Compound 3(26-*0*-β-D-glucopyranose-3β, 26-diol-(25R)-Δ^{5,20(22)}-3-*0-*{[α-L-(25R)-pyranorhamnose(1→4)]-β-D-glucopyranoside}): White powder. ESI-MS *m*/*z*: 907 [M+Na]⁺, 885 [M+H]⁺, 723 [M-glc+H]⁺, 577 [M-glc-rha+H]⁺, ¹H NMR (600MHz, C₅D₅N): δ1.62 (3H, s, CH₃-21), 1.02 (3H, d, CH₃-27, *J*=6.5 Hz), 0.88 (3H,s, CH₃-19), 0.70(3H, s, CH₃-18) present 4 signals of methyl proton of the aglycone of dioscin. δ1.77(3H,d, *J*=6.0 Hz) shows the signal at the end of methyl proton of rhamnose. The ¹³C NMR data of compound 3 see table 4. The aglycone part is the same as that of compound 8. The NMR data of compound 3 is in accordance with those reported in reference 5.

Compound 2 (pseudoprotodioscin): White powder. ESI-MS *m*/*z:* 1053[M+Na]⁺, 723[M-rha-glc+H]⁺, 577[M-rha-glc-rha+H]⁺, 415[M-rha-glc-rha-glc+H]⁺, 1029[M-H]⁻, 883[M-rha-H]⁻; ¹H NMR(400 MHz, C₅D₅N): δ1.61(3H,s,CH₃-21), 1.03(3H, s, CH₃-19), 0.99(3H, d, CH₃-27, *J*=6.8 Hz), 0.70(3H, s, CH₃-18) present 4 signals of methyl proton of the aglycone of dioscin. δ1.74(3H, d, *J*=7.2 Hz), 1.60 (3H, d, *J*=7.7 Hz) present 2 signals of methyl proton of rhamnose, respectively. δ6.37(1H, br, s), 5.83(1H, br, s) shows these two rhamnoses are of a type. δ4.92(1H, d, *J*=6.9 Hz), 4.81(1H, d, *J*=7.7 Hz) shows these two glucoses are of β type. ¹³C NMR data of compound 2 (see Table 4) is in accordance with those reported in reference 5.

Compound 1 (protodioscin): For methods for separation and identification, consulted in citation of *Bogang Li; Zhengzhi Zhou. Traditional Chinese Drug Research & Clinical Pharmacology 1994*, *13(2)*: 75-76 can be taken for reference.

**Table 1 ¹³C NMR Data Of Compound 2-9 (Solvent: C₅D₅N)**

| C | 9 | 8 | 7 | 6 | 5 | 4 | 3 | 2 |
|---|---|---|---|---|---|---|---|---|
| 1 | 37.3 | 37.4(t) | 37.4(t) | 37.5(0) | 37.5(t) | 37.5(t) | 37.5(t) | 37.5(t) |
| 2 | 29.9 | 30.2(t) | 30.2(t) | 30.1(t) | 30.1(t) | 30.2(t) | 30.2(t) | 30.1(t) |
| 3 | 77.7 | 78.6(d) | 78.3(d) | 78.2(d) | 77.8(d) | 78.3(d) | 78.3(d) | 78.0(d) |
| 4 | 38.4 | 39.3(t) | 39.3(t) | 38.9(t) | 38.9(t) | 39.3(t) | 38.9(t) | 38.9(t) |
| 5 | 140.5 | 140.9(s) | 140.9(s) | 140.8(s) | 140.8(s) | 140.9(s) | 140.8(s) | 140.8(s) |
| 6 | 121.6 | 121.7(d) | 121.7(d) | 121.7(d) | 121.7(d) | 121.7(d) | 121.7(d) | 121.8(d) |
| 7 | 32.2 | 32.2(t) | 32.2(t) | 32.3(t) | 32.3(t) | 32.3(t) | 32.4(t) | 32.4(t) |
| 8 | 31.2 | 31.6(d) | 31.6(d) | 31.6(d) | 31.6(d) | 31.4(d) | 31.4(d) | 31.4(d) |
| 9 | 50.1 | 50.3(d) | 50.3(d) | 50.3(d) | 50.3(d) | 50.3(d) | 50.2(d) | 50.3(d) |
| 10 | 36.9 | 37.0(s) | 37.0(s) | 37.1(s) | 37.1(s) | 37.0(s) | 37.1(s) | 37.1(s) |
| 11 | 21 | 21.1(t) | 21.1(t) | 21.0(t) | 21.1(t) | 21.1(t) | 21.2 (t) | 21.2(0 |
| 12 | 39.4 | 39.9(t) | 39.8(t) | 39.8(t) | 39.8(t) | 39.6(t) | 39.6(s) | 39.6(t) |
| 13 | 43.2 | 40.4(s) | 40.4(s) | 40.4(s) | 40.4(s) | 43.4(s) | 43.4(s) | 43.4(s) |
| 14 | 54.7(d) | 56.6(d) | 56.6(d) | 56.6(d) | 56.6(d) | 54.9(d) | 54.9(d) | 54.9(d) |
| 15 | 34.3 | 32.2(t) | 32.2(t) | 32.2(t) | 32.2(t) | 34.4(t) | 34.5(t) | 34.5(t) |
| 16 | 84.3 | 81.1(d) | 81.1(d) | 81.1(d) | 81.1(d) | 84.4(0) | 84.4(d) | 84.4(d) |
| 17 | 64.3 | 62.9(d) | 62.9(d) | 62.8(d) | 62.9(0) | 64.5(d) | 64.4(0) | 64.5(d) |
| 18 | 13.9 | 16.3(q) | 16.3(q) | 16.3(q) | 16.3(q) | 14.1(q) | 14.1(q) | 14.1(q) |
| 19 | 19.2 | 19.4(q) | 19.4(q) | 19.3(q) | 19.3(q) | 19.4(q) | 19.4(q) | 19.4(q) |
| 20 | 103.4 | 42.0(d) | 41.9(d) | 41.9(d) | 41.9(0) | 103.5(s) | 103.6(s) | 103.5(s) |
| 21 | 11.6 | 15.0(q) | 15.0(q) | 14.9(q) | 15.0(q) | 11.8(q) | 11.8(q) | 11.8(q) |
| 22 | 152.2 | 109.2(s) | 109.3(s) | 109.2(s) | 109.2(s) | 152.4(s) | 152.3(s) | 152.4(s) |
| 23 | 31.2 | 31.8(t) | 31.8(t) | 31.8(t) | 31.8(t) | 31.4(t) | 31.4(t) | 31.4(t) |
| 24 | 23.5 | 29.2(t) | 29.2(t) | 29.2(t) | 29.2 (t) | 23.7(t) | 23.6(t) | 23.7(t) |
| 25 | 33.7 | 30.6(d) | 30.6(0) | 30.5(0) | 30.5(d) | 33.5(d) | 33.5(d) | 33.4(0) |
| 26 | 74.7 | 66.8(t) | 66.8(t) | 66.8(t) | 66.8(t) | 74.9(t) | 74.9(t) | 74.9(t) |
| 27 | 17.1 | 17.3(q) | 17.3(q) | 17.2(q) | 17.2(q) | 17.3(q) | 17.3(q) | 17.3(q) |
| C3 | | | | | | | | |
| Sugar | | | | | | | | |
| Art | | | | | | | | |
| Glyc 1' | 99.7 | 102.6(d) | 102.7(d) | 100.3(d) | 100.2(d) | 102.7(0) | 100.3(d) | 100.2(d) |
| (inner)2' | 76.8 | 75.4(0) | 75.5(d) | 79.6(d) | 78.1(d) | 75.5(d) | 79.6(d) | 78.6(d) |
| 3' | 89.3 | 78.5(d) | 76.7(d) | 77.8(d) | 76.8(d) | 76.7(0) | 77.8(d) | 76.8(d) |
| 4' | 71.3 | 71.70) | 78.2(d) | 71.6(d) | 78.7(d) | 78.5(0) | 71.7(d) | 78.6(d) |
| 5' | 78.3 | 78.1(d) | 77.1(d) | 77.9(0) | 77.9(d) | 77.1(d) | 77.7(d) | 77.7(d) |
| 6' | 62.2 | 62.9(t) | 61.5(t) | 62.6(t) | 61.3(t) | 61.5(t) | 62.6(t) | 61.3(t) |
| rha 1" | 102 | | 102.4(d) | | 102.0(d) | 102.4(d) | | 102.O(O) |
| (1→4)2" | 72.5 | | 72.8(d) | | 72.4(d) | 72.8(d) | | 72.5(d) |
| 3" | 72.2 | | 72.6(d) | | 72.8(d) | 72.6(d) | | 72.8(0) |
| 4" | 73.9 | | 74.0(d) | | 74.1(d) | 74.0(d) | | 74.1(d) |
| 5" | 69.3 | | 70.3(d) | | 69.4(d) | 70.3(d) | | 69.5(d) |
| 6" | 18.5 | | 18.5(q) | | 18.6(q) | 18.5 (q) | | 18.6(q) |
| rha 1'" | 104.3 | | | 102.0(d) | 102.9(d) | | 102.O(O) | 102.9(d) |
| (1→2)2"' | 74.7 | | | 72.5(0) | 72.4(d) | | 72.5(d) | 72.5(d) |
| 2"' | | | | | | | | |
| 3"' | 78.3 | | | 72.8(d) | 72.7(d) | | 72.8(d) | 72.7(d) |
| 4"' | 71.3 | | | 74.1(d) | 73.8(d) | | 74.1(0) | 73.9(d) |
| 5'" | 77.4 | | | 69.4(d) | 70.4(0) | | 69.5(d) | 70.4(d) |
| 6"' | 62.2 | | | 18.6(q) | 18.4(q) | | 18.7(q) | 18.4(q) |
| C-26 | | | | | | | | |
| Sugar | | | | | | | | |
| Art | | | | | | | | |
| glc 1"" | 104.7 | | | | | 104.9(d) | 104.9(d) | 104.9(d) |
| 2"" | 75 | | | | | 75.2(d) | 75.2(d) | 75.2(d) |
| 3"" | 78.5 | | | | | 78.6(d) | 78.6(d) | 78.5(0) |
| 4"" | 71.5 | | | | | 71.7(0) | 71.7(d) | 71.7(d) |
| 5"" | 78.4 | | | | | 78.2(d) | 78.3(0) | 77.9(d) |
| 6"" | 62.6 | | | | | 62.9(0) | 62.8(0) | 62.8(0) |

With the above methods, all compounds in the pharmaceutical composition of the present invention can be separated and identified.

### Example 5 Quality control of the pharmaceutical composition of the present invention

9 reference substances that have been confirmed with their structures were introduced in way of mixed sampling for HPLC assay under chromatographic condition as follows: chromatographic column: Alltima C18 4 6×250 mm, 5 µm, gradient elution; determined with an evaporative light scattering detector; drift tube temperature: 100 °C; gas flow rate: 2.0L / min. The chart of gradient elution is as follows:

**Table 2 Gradient elution chart**

| | 0(min) | 30(min) | 50(min) | 60(min) | 80(min) |
|---|---|---|---|---|---|
| Acetorlitrile | 15% | 40% | 40% | 100% | 100% |
| Water | 85% | 60% | 60% | 0 | 100% |

Chromatogram is shown in Figure 1.

After HPLC assay for the compositions obtained in example 1, 2 and 3, chromatograms are shown in figure1, figure2, figure3. Two points external standard method of logarithmic equation was applied for calculation of the percentage of the weight of compound I, II and III, as shown in Table 3.

**Table 3 Percentage of compound I,II, and III by weight in total steroidal saponins.**

| | Compound (I) % | Compound (II)% | Compound (III)% |
|---|---|---|---|
| Example 1 | 59.8 | 4.7 | 5.7 |
| Example 2 | 63.8 | 4.8 | 6.8 |
| Example 3 | 58.4 | 6.8 | 9.5 |

After follow-up determination of 20 batches of the samples of the pharmaceutical composition of the present invention, it was demonstrated that contents for three compounds thereof were stable relatively, and thus they were made as the indexes for quality control and had rather strong controllability. As shown in Table 4:

**Table 4 Percentage of each steroidal saponin of the composition of the present invention by weight to the total steroidal saponins**

| Batch No. | Protodioscin (%) | Pseudoprotodioscin(%) | Pseudoprotogracillin(%) | Compound 3 | Dioscin (%) |
|---|---|---|---|---|---|
| 1 | 2.1 | 60.6 | 6.2 | 8.1 | 7.8 |
| 2 | 0.8 | 60.2 | 5.3 | 9.4 | 9.3 |
| 3 | 1.6 | 61.8 | 5.9 | 8.4 | 7.0 |
| 4 | 3.9 | 58.4 | 6.8 | 6.8 | 9.5 |
| 5 | 1.5 | 66.6 | 6.8 | 1.6 | 6.8 |
| 6 | 9.8 | 63.0 | 4.7 | 0.86 | 6.2 |
| 7 | 7.7 | 63.8 | 4.8 | 1.0 | 6.8 |
| 8 | 3.0 | 65.7 | 5.6 | 0.9 | 8.4 |
| 9 | 8.6 | 63.2 | 4.9 | 1.1 | 6.3 |
| 10 | 6.3 | 64.8 | 5.4 | 1.1 | 6.3 |
| 11 | 11.2 | 61.2 | 5.2 | 1.2 | 5.8 |
| 12 | 9.8 | 60.6 | 6.1 | 1.2 | 7.2 |
| 13 | 8.7 | 62.2 | 5.3 | 1.1 | 7.2 |
| 14 | 2.2 | 67.7 | 5.6 | 1.1 | 6.5 |
| 15 | 7.7 | 63.5 | 5.0 | 1.1 | 6.8 |
| 16 | 13.4 | 59.8 | 4.7 | 1.4 | 5.7 |
| 17 | 2.0 | 68.6 | 5.8 | 1.0 | 5.4 |
| 18 | 3.3 | 66.9 | 4.9 | 1.7 | 6.4 |
| 19 | 3.2 | 66.0 | 4.7 | 1.6 | 8.1 |
| 20 | 6.5 | 62.4 | 4.5 | 1.5 | 7.2 |
| 21 | 10.1 | 60.2 | 5.3 | 1.3 | 7.1 |
| 23 | 6.7 | 65.8 | 4.5 | 0.9 | 5.7 |
| 24 | 2.9 | 68.1 | 5 0 | 1.1 | 5.9 |

It was proved in the above detection that the pseudoprotodioscin (□), pseudoprotogracillin (II), dioscin (III) were all in constant proportion in the pharmaceutical composition of the present invention. By controlling directly the contents of these three compounds, the object for controlling the quality of medicine of the present invention is obtainable. In different raw materials and procedures to extract the same, the pseudoprotodioscin (I) and the protodioscin (IV) in the above table could converse to each other. Nevertheless, tests proved that in the same batch of raw material and with the same method for preparation, the contents of pseudoprotodioscin (I) and the protodioscin (IV) were basically constant and so were the contents of pseudoprotogracillin (II) and protogracillin (V). One of the compounds or two of the same with higher contents could be used as the one for quality control. That is to say the pseudoprotodioscin (I) and/or protodioscin (IV), pseudoprotogracillin (II) and/or protogracillin (V), dioscin (III) were all in constant proportion and by directly controlling the contents of these three compounds, the object for controlling the quality of the medicine of the present invention could be achieved.

### Example 6 Preparation of the capsules containing the pharmaceutical composition of the present invention

Ingredients of the capsule (for 1000 capsules):

| | |
|---|---|
| Total steroidal saponin (obtained in example 2) | 100 g |
| Starch | 150 g |

Steriodal saponin and starch were taken according to the proportion as specified above. After mixing, the drug was encapsuled, each containing 250 mg, and prescribed orally for treatment of cerebrocardiovascular diseases at the dosage of 1- to 2 caps, 3 times daily for two months.

The steroidal saponin contained in each capsule shall not be less than 65.0 mg, being calculated by the content of discon.

### Example 7 Preparation of tablets

Ingredients of tablet (for 1000 tablets):

| | |
|---|---|
| Total steroidal saponin (obtained as in example 2) | 100 g |
| HPMC _{LV100} | 30g |
| Lactose | 70 g |
| Magnesium stearate | 1 g |

The total steroidal saponin, HPMC and lactose were mixed up and made with 75% of ethanol as the adhesive into wet grains, which were screened with 22 M. After being dried at 50°C for 3 hrs, the grains were again screen at 22 M before being added with magnesium stearate and made into tablets, each weighing 0.15 g. The medicine can be prescribed orally for cerebrocardiovascular diseases at the dosage of 1- to 2 tabs, 3 times daily for two months.

Each tablet contains steroidal saponin not less than 65.0 mg when calculated by content of dioscin.

### Example 8: Preparation of injections

Ingredients for injection (for 1000 ml):

| | |
|---|---|
| Total steroidal saponin | 50 g |
| Tween 80 | 10 ml |
| Sodium chloride | 8 g |

Total steroidal saponin was added with 10% Na₂CO₃ with pH value adjusted to 7.0~ to 7.5. After refrigeration and filtration, tween 80 and NaCl were added and injection water was added to 1000 ml. After being filtered with G₃ sintered filter funnel (Glass), and sub-packed and then filled and sealed. The final product was obtained after wet sterilization at 100°C. The injection can be prescribed hypodermically for treatment of cerebrocardiovascular diseases at the dosage of 1~ to 2 ml, twice daily for two months.

Each contains steroidal saponin not less than 65.0 mg, as calculated with content of dioscin.

Soft capsules and dripping pills of the same could be prepared according to routine method with addition of substrate in common usage.

The advantageous results of the pharmaceutical composition of the present invention were proved in following pharmacodynamic tests.

### Test 1 Effect of pseudoprotogracillin (II) on acute myocardial infarction in rats:

Pseudoprotogracillin (II) obtained in example 4 was applied in relevant pharmacological tests as follows:
Wistar rats, each weighing 180~ to 220 g, were randomized into 3 groups, namely control group, pseudoprotogracillin group of high dose and pseudoprotogracillin group of low dose, each of which had 12 rats. The rats in the control group were administered by gastric infusion of distilled water. The other rats in test groups were administered by gastric infusion of the relevant medicinal liquid (prepared with distilled water), according to the high dose (3 mg/kg) or low dose (1.5 mg/kg) listed in following table for each group, respectively. Infusion was performed once a day and continued for one week. 1 hour after last administration, urethane of 1 g/kg was given to the rats for abdominal anesthesia. The animal was fixed on its back and its skin was cut open along the midline of its sternum, along the left edge of which an incision was made for the heart to be squzeezed out quickly. Ligation was immediately made at the root of the left anterior descending branch of its coronary artery before putting the heart back into its thorax, which was then closed by suture and spontaneous breathing was restored. The test was ended 3 hours after ligation and the heart was sliced transversely below the line of ligation. 5 slices were blue-stained with nitrotetrazolium and an multiple color pathological analyzer (MPIAS-500) was applied to measure the areas of normal myocardium and infarct myocardium at a fixed imaging distance and to observe the degree of myocardial infarction. After being treated statistically (t test), the results are shown in Table 5.

**Table 5 Effect of pseudoprotogracillin (II) on myocardial infarction induced by ischemia of myocardium (X±s)**

| Group | Dosage mg/kg | Area of infarction mm² | Weight of infarction g | Percentage of infarct area to ventricle (%) | Percentage of infarct area to the heart (%) |
|---|---|---|---|---|---|
| Control | | 112.23±9.46 | 0.301±0.055 | 32.9±3.1 | 128.6±1.5 |
| High dose | 3 | 380.56±7. 69** | 0.227±0.032** | 24.5±2.6** | 20.74±1.9** |
| Low dose | 1.5 | 91.3±11.49* | 0.241±0.045* | 28.1±1.9* | 25.2±2.3* |

| | | | | | |
|---|---|---|---|---|---|
| In comparison with control group: *P<0.05 **P<0.01 | | | | | |

The results in Table 4 demonstrated that in the groups treated with compound (II) at both high dose or low dose, the area and weight of infarct myocardium, the percentages of infarct area to ventricle and to the whole heart are significantly lower in comparison with the control group (P<0. 01 and P<0. 05, respectively), and present a dose-effect relationship, indicating the novel compound pseudoprotogracillin (II) of the present invention can minimize the extent of myocardial infarction, reduce the area of infarction and weight of infarct area and has obvious action to protect myocardium.

The above pharmacodynamical test does not only demonstrate that the compound pseudoprotogracillin (II) in the pharmaceutical composition of the present invent is a novel compound, but also proves strongly that the new compound can be prepared independently into an agent with definite pharmacodynamic action.

### Test 2 Effects of the pharmaceutical composition of the present invention on angina, blood lipids and platelet aggregation

1084 patients with thoracic obstruction (angina) were clinically observed in a way of random, double blind and control. They were prescribed at random with A1, A2, A3, A4, Fufangdanshen Tablets and isosorbide dinitrate.

All cases to be administered with the drugs had gone through tests of proportionality (comparability) in aspects of genders, ages, courses of disease and complications. The results proved that the patients in groups taking A1, A2, A3 and A4 capsules and in group taking Fufangdanshen Tablet and isosorbide dinitrate had comparability with no obvious differences in their genders, ages, courses of disease and complications.

6 drugs labeled as A1, A2, A3, A4, B and C were all encapsuled with same appearance. Five of the drugs were taken orally, 1 capsule, 3 times daily. These 5 drugs had 8 weeks as one therapeutic course. During the period for administration of these 6 drugs, all patients stopped taking any medicine that might have effects on their coronary blood flow, blood lipids and blood pressure. In case of emergency, the said medicine could be applied temporarily, but once the condition turned better, the said medicine should be withdrawn and the dose should be recorded.

Unblinding: A1, A2 and A3 were the capsules comprising the pharmaceutical composition obtained in example 1, example 2 and example 3 and prepared with the method in example 6. A4 was the capsules comprising the composition extracted with old procedures and prepared with the method in example 6. The content of total steroidal saponin in each capsule of A1, A2, A3 and A4 was the same. The dose was 200 mg each time, 3 times daily. B was Fufangdanshen Tablet, the dose of which was 3 tabs, 3 times daily. C was the drug of isosorbide dinitrate and the dose for it was 5 mg, 3 times daily.

All patients should be withdrawn from the medicines that have effects on coronary heart disease 3 days before receiving testing drugs, and should have their blood and urine tests, hepatic and renal function exams, and blood lipids, rest ECG and load test analyzed. After treatment, all of the above said indexes should be rechecked.

During treatment, all medicines to reduce blood pressure and blood fat and to expand coronary artery should be withdrawn. Only those patients with frequent angina and serious cardiac arrhythmias, nitroglycerin drugs could be prescribed temporarily, and dosage and time of stoppage should be recorded.

Criteria to assess therapeutic effects: *Standard for Therapy of Coronary Heart Disease and Angina and Electrocardiogram* amended in 1979 in the National Research Symposium on Treatment and Prevention of Coronary Heart Disease, Angina and Arrhythmia with Combination of Traditional Chinese Medicine and Western Medicine was adopted for therapeutic assessment.

Results are shown in table 6 and 7:

**Table 6 Comparison of the effects of 5 drugs on angina**

| group | Total cases | Obvious % effective | | Effective | % | No effective | % | Total effective% |
|---|---|---|---|---|---|---|---|---|
| Algroup | 178 | 72 | 40.4 | 86 | 48.3 | 20 | 10.1 | 89.9 |
| A2 group | 179 | 74 | 41.3 | 88 | 49.2 | 17 | 9.5 | 90.5 |
| A3 group | 178 | 71 | 39.9 | 90 | 50.6 | 17 | 9.6 | 90.4 |
| A4 group | 258 | 99 | 38.4 | 117 | 45.3 | 42 | 16.3 | 83.7 |
| Fufangdanshen Tablets | 123 | 34 | 27.6 | 44 | 35.8 | 45 | 36.6 | 63.4 |
| isosorbide dinitrate | 168 | 37 | 22.0 | 80 | 47.6 | 51 | 30.4 | 69.6 |

By Ridit analysis, all p values in A1, A2 and A3 groups were less than 0.01 in comparison with those in B group and in C group, respectively. By *X²* analysis, A1, A2 and A3 group presented an obvious effective rate in comparison with B group, P<0.05; A1, A2 and A3 group presented an obvious effective rate in comparison with C group, P<0.01. The results demonstrated that the therapeutic effects of the capsules made from the composition obtained in example 1, example 2 and example 3 of the present invention are better than those of Fufangdanshen Tablet (B) and isosorbide dinitrate (C). The total effective rates to relieve angina were 89.9%, 90.5 % and 90.4% respectively and definitely, proving that an effective and stable pharmacodynamic actions were achieved by controling of 3 components.

In comparison with routine method, the preparation procedures of the present invention decrease cost by more than 35% and increase yield by 13%. With same amount of raw material, the pharmacodynamic action increased by 5% when the pharmaceutical composition of the present invention was adopted in clinical observation of 258 cases.

### Effect on ischemia of myocardium

In 8 weeks for a course of treatment, the comparison of the pharmaceutical composition of the present invention is shown in Table 7.

**Table 7 Comparison of therapeutic effects of 6 drugs on myocardial ischemia**

| group | Total cases | Obvious effective | % | Effective | % | No effective | % | Total effective% |
|---|---|---|---|---|---|---|---|---|
| A1 group | 132 | 41 | 31.1 | 31 | 23.5 | 60 | 45.4 | 55.5 |
| A2 group | 132 | 42 | 31.8 | 32 | 24.2 | 58 | 43.9 | 56.0 |
| A3 group | 131 | 41 | 31.1 | 31 | 23.7 | 59 | 45.0 | 54.8 |
| A4 group | 258 | 76 | 29.4 | 58 | 22.5 | 124 | 48.1 | 51.9 |
| Fufangdanshen tablets | 57 | 10 | 17.5 | 12 | 21.1 | 35 | 61.4 | 38.6 |
| isosorbide dinitrate | 85 | 25 | 29.4 | 20 | 23.5 | 38 | 44.7 | 52.9 |

By Ridit analysis, the therapeutic effects on ischemia of myocardium in A1, A2 and A3 groups had no obvious differences with that in isosorbide dinitrate group, respectively (all P>0.05), but surpassed that in Fufangdanshen Tablet group (all P<0.01). By *X²* analysis, the obvious effective rates of A1, A2 and A3 groups were higher than that in Fufangdanshen group, P<0.05, but with no differences with that in isosorbide dinitrate group, all p<0.05, indicating that capsules made from the pharmaceutical composition of the present invention obtained as in example 1, example 2 and example 3 had better therapeutic effects on angina than that of Fufangdanshen Tablet (B), and had the equivalent effect as that in isosorbide dinitrate group (C). The total effects on ST-T segments of the rest ECG were 55.5%, 56.0% and 54.8% respectively, proving the effects were definite and effective and stable pharmacodynamic actions had been achieved by controlling 3 components. The effects of the pharmaceutical composition of the present invention were 5% higher than that prepared with old procedures.

### Effect on blood lipids

Among 164 patients with hypercholesteremia and 162 patients with hypertriglyceridemia before treatment, 78 patients with hypercholesteremia turned normal and so did 87 patients with hypertriglyceridemia after treatment. The cholesterol in their blood dropped from 6.8±1.1 mmol/L before treatment to 6.3±1.1 mmol/L after treatment. The triglyceride in their blood dropped from 2.4±0.8 mmol/L before treatment to 1.9±0.7 mmol/L after treatment. The differences were of great significance (P<0.01) by t test.

### Effect on platelet aggregation

The pharmaceutical composition had an obvious effect on platelet aggregation induced by denosine diphosphate (ADP) and adnephrin. ADP decreased the rate of platelet aggregation from 68%±15% before being treated with the composition to 60 %±15% after being treated composition; adnephrin dropped the aggregation rate from 73%±15% before being treated with the composition to 64%±12% after being treated, indicating a difference of great significance after t test (P<0.01).

Improvement of the pharmaceutical composition of the present invention on clinical symptoms of patients with coronary heart disease

**Table 8 Improvement of main symptoms before and after treatment with the capsules comprising the pharmaceutical composition of the present invention**

| | Total cases | Obviously effective | effective | No effect | Worsen | Rate of obvious effect(%) | Total effective rate (%) |
|---|---|---|---|---|---|---|---|
| chest distress | 244 | 112 | 212 | 12 | 0 | 46 | 87 |
| cardiopalmus | 225 | 123 | 193 | 31 | 1 | 55 | 86 |
| short breath | 263 | 104 | 203 | 59 | 1 | 40 | 77 |
| acratia | 226 | 68 | 155 | 69 | 2 | 30 | 68 |
| dizziness | 206 | 83 | 147 | 58 | 1 | 40 | 71 |
| headache | 168 | 58 | 117 | 49 | 2 | 34 | 70 |

### Adverse effects

Among 825 cases in this test, 5 had dizziness during the period of administration, 9 had stomach or abdominal discomfort, 2 had headache and 1 had hectic. All of the patients with above-mentioned adverse effect were not affected in their treatment and their routine blood and urine tests, hepatic and renal function exams presented normal before and after treatment.

It is well illustrated by the above-mentioned preparation procedures, structural determinations, quantified control and pharmacodynamic tests that simultaneous control of the three compounds of the pharmaceutical composition of the present invention, namely pseudoprotodioscin (I), pseudoprotogracillin (II) and dioscin (III), and effective quality control thereof can increase the pharmacodynamic action and stability of the drugs made thereof with minimized dosage and convenient usage thereof.

### References:

1. P.K AGRAWAL, D.C JAIN, R.K GUPTA et al, CARBON-13 NMR SPECTROSCOPY OF STEROIDAL SAPOGENINS AND STEROIDAL SAPONINS. Phytochemistry, 1985, 24(11): 2479-2496.
2.Chenji Xu. Research and development of medicinal plants for steroid hormone drugs in Chinese cinnamonvine resourses. Sichuan Press of Science and Technology 2000.
3. Mei Dong et al. TWO novel furostanol saponins from the rhizomes Of Dioscorea panthaica Prain et Burkill and their cytotoxic activity. Tetrahedron, 2001, 57, 501-506.
4. Zhongliang Zhou, RitaAquino, Francesco et al. Oligofurostanosides from Asparagus cochinchinensis. Planta Medica. 1988, 50(4): 344-346.
5. Mei Dong et al. Separation and identification of steroidal saponins from Dioscorea panthaica Prain et Burkill. ACTA MEDICA SINICA 2001, 36(1): 42-45.
6. Shuhu Du et al. Separation and identification of steroidal saponins from Dioscorea nipponica Makino. ACTA MEDICA SINICA 2002, 37(4): 267-270.

## Claims

1. A pharmaceutical composition containing steroidal saponins, comprising:
5 to 20 parts by weight of furostanol saponin, said furostanol saponin being pseudoprotodioscin (I) and being represented by general formula A in which R₁ = 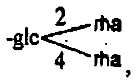
1 to 3 parts by weight of furostanol saponin, said furostanol saponin being pseudoprotogracillin (II) and being represented by general formula A in which R₁ = 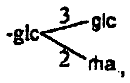
1 to 5 parts by weight of spirostanol saponin or sapogenin, said spirostanol saponin or sapogenin being dioscin (III) and being represented by general formula C in which R₃ = 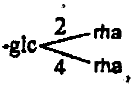 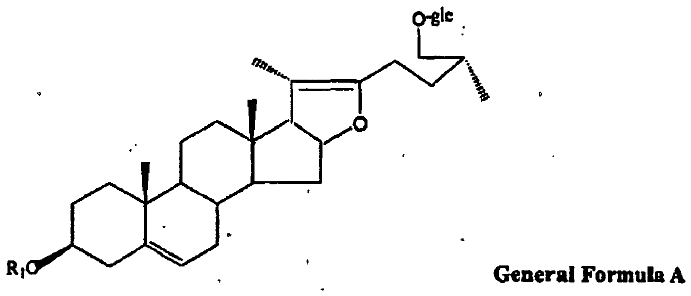 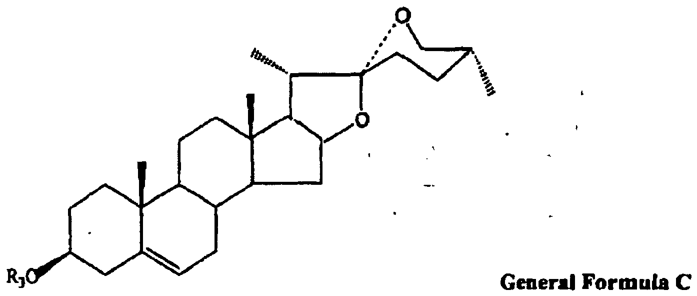 said steroidal saponins being originated from the extracts of *Dioscorea panthaica* Prain et Burkill and *Dioscorea nipponica* Makino, both belonging to species of Dioscorea Dioscoreaccae, and
said extract being prepared by the method comprising the steps of:
a. taking rootstocks of *Dioscorea panthaica* Prain et Burkill and *Dioscorea nipponica* Makino, or the rootstocks of freshly-collected *Dioscorea panthaica* Prain et Burkill and *Dioscorea nipponica* Makino as raw material;
crushing and slicing the rootstocks;
extracting with one or more than one kinds of solvents selected from a group consisting of water, methanol, ethanol, n-butanol and other low-fat alcohols, the amount of said solvent shall be 24 to 48 times of the raw material;
b. cooling and filtering the extract prepared in step a;
passing the filtrate through an absorbent resin column,
abandoning the effluent and washing with water till the effluent turns colorless; abandoning the rinsing water;
c. eluting the absorbent resin column that is washed with water in step b with one or more than one kinds of solvent selected from a group consisting of ethanal, methanol, acetone, 50 to 90 % ethanol, 30 to 80% hydrous methanol and 60 to 95% hydrous acetone;
collecting and condensing the effluent;
d. adding 60 to 95% alcohol to the concentrated solution obtained in step c for alcohol precipitation, filtering and collecting the filtrate;
condensing and drying the filtrate to obtain product.

2. The pharmaceutical composition according to claim 1, wherein it comprises:
12 to 18 parts by weight of pseudoprotodioscin (I),
1 part by weight of pseudoprotogracillin (II), and
1.2 to 2.5 parts by weight dioscin (III).

3. The pharmaceutical composition according to claim 1, wherein in said extract, the content of total steroidal saponin is no less than 65% (w/w) when calculated by dioscin.

4. The pharmaceutical composition according to claim 1 or 2, wherein in said extract, the total content of three kinds of steroidal saponins, pseudoprodioscin (I), pseudoprotogracillin (II) and dioscin (III), is no less than 50% (w/w) of the content of total steroidal saponins.

5. The pharmaceutical composition according to claim 1, 3 or 4, wherein said extract has the HPLC fingerprint as shown in Fig 1, in which the characteristic peaks of said HPLC fingerprint are as follows:
the retention time for pseudoprotodioscin (I): 28.27 min;
the retention time for pseudoprotogracillin (II): 29.5 min; and
the retention time for dioscin (III): 57.10 min;
the chromatographic conditions of HPLC are as follows :
chromatographic column: Alltima C18 4.6×250 mm, 5µm:
gradient elution;
determined with an evaporative light scattering detector;
drift tube temperature: 100°C ; and
gas flow rate: 2.0L / min.

6. The pharmaceutical composition according to claim I, wherein in step b, it further includes a step of decompressing condensation before cooling and filtering when the extract contains methanol, ethanol, n-butanol or other low-fat alcohols.

7. The pharmaceutical composition according to any of claims to 6, wherein it comprises said steroidal saponins or the extracts of *Dioscorea panthaica* Prain et Burkill and *Dioscorea nipponica* Makino as an active ingredient and pharmaceutically acceptable adjuvants.

8. The pharmaceutical composition according to claim 7, wherein said pharmaceutical composition is in form of tablet, capsule, soft capsule, grain, oral liquor, dripping pill or injection.

9. A method for preparing the pharmaceutical composition according to any of claims 1 to 8, wherein it includes the following steps:
taking by weighing furostanol saponins of general formula A and spirostanol saponin of general formula C, mixing them with the ratio by weight of 5 to 20 parts of furostanol saponin, said furostanol saponin being pseudoprotodioscin (I), of 1 to 3 parts by weight of furostanol, saponin, said furostanol saponin being pseudoprotogracillin (II), and 1 to 5 parts of spirostanol saponin or sapogenin, said spirostanol saponin or sapogenin being dioscin (III); adding pharmaceutically acceptable adjuvants to obtain the pharmaceutical composition.

10. A method for preparing the pharmaceutical composition according to any of claims 1 to 8, wherein it includes the following steps:
a. taking rootstocks of herbs *Dioscorea panthaica* Prain et Burkill and *Dioscorea wripponlea* Makino, or the rootstocks of freshly-collected *Dioscorea panthaica* Prain et Burkill and *Dioscorea nipponica* Makino as the raw material, slicing rootstocks or smashing them, extracting with one or more than one kinds of solvent selected from a group consisting of water, methanol, ethanol, n-butanol and other low-fat alcohols, the amount of which shall be 24 to 48 times of the raw material;
b. cooling and filtering the extract prepared in step a, passing the filtrate through absorbent resin column, abandoning the effluent and washing with water till the effluent turns colorless; abandoning the rinsing water;
c. eluting the absorbent resin column that was washed with water in step b with one or more than one kinds of solvent selected from a group consisting of ethanol, methanol, acetone, 50% to 90% ethanol, 30% to 80% hydrous methanol and 60% to 95% hydrous acetone, collecting and condensing the effluent;
d. adding 60% to 95% alcohol to the concentrated solution obtained in step c for alcohol precipitation, collecting filtrate after filtered;
e. condensing and drying the filtrate in step d, adding pharmaceutically acceptable adjuvants to obtain the pharmaceutical composition.

11. The method according to claim 10, wherein in step b, it further includes a step of decompressing condensation before cooling and filtering when the extract contains methanol, ethanol, n-butanol or other low-fat alcohols.

12. A use of the pharmaceutical composition of claims 1 to 8, in preparing the pharmaceutical for treatment and prevention of cerebrocardiovascular diseases.

13. The use according to claim 12, wherein said diseases are coronary heart disease, angina, myocardial infarction, arrhythmia, hyperlipermia or ischemic cerebrovascular diseases.

## Patentansprüche

1. Pharmazeutisch Zusammensetzung enthaltend steroidale Saponine, umfassend:
5 bis 20 Gewichtsteile Furostanol-Sapanin, wobei das Furostanol-Saponin Pseudoprotodioscin (I) ist und durch die allgemeine Formel A wiedergegeben wird, worin R₁ = 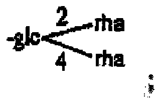
1 bis 3 Gewichtsteile Furostanol-Saponin, wobei das Furostanol-Saponin Pseudoprotogracillin (II) ist und durch die allgemeine Formel A wiedergegeben wird, worin R₁ = 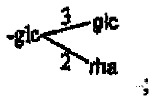 und
1 bis 5 Gewichtsteile Spirostanol-Saponin oder Sapogenin, wobei das Spirostanol-Saponin oder Sapogenin Dioscin (III) ist und durch die allgemeine Formel C wiedergegeben wird,
worin R₃ = 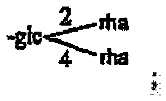 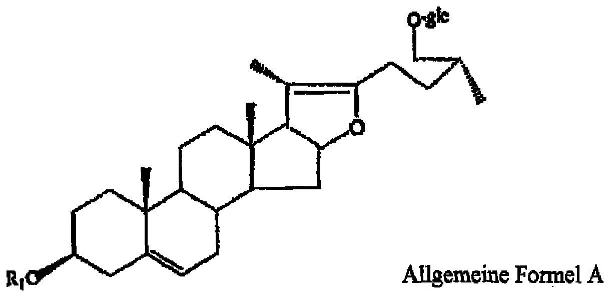 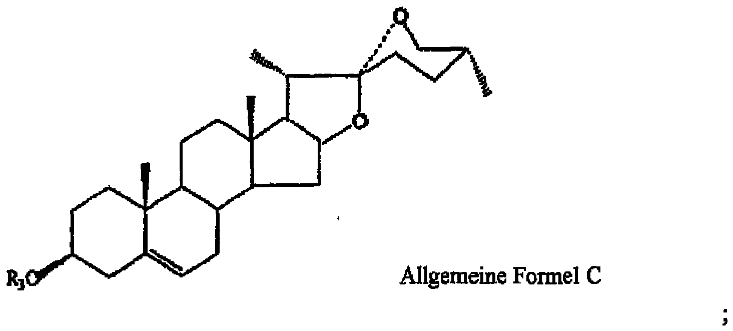 wobei die steroidalen Sapanine den Extrakten von *Dioscorea panthaica* Prain et Burkill und *Dioscorea nipponica* Makino entstammen, welche beide der Gattung Dioscorea innerhalb der Familie der Dioscoreaceae angehören, und
wobei der Extrakt durch das Verfahren hergestellt wird, welches die folgenden Schritte umfasst:
a. Gewinnen der Wurzelstöcke von *Dioscorea panthaica* Prain et Burkill und *Dioscorea nipponica* Makino oder der Wurzelstöcke von frisch gesammelten Pflanzen von *Dioscorea panthaica* Prain et Burkill und *Dioscorea nipponica* Makino als Ausgangsmaterial;
Zerdrücken und Zerteilen der Wurzelstöcke; und
Extrahieren mit einem oder mehreren Lösungsmitteln, gewählt aus der Gruppe, bestehend aus Wasser, Methanol, Ethanol, n-Butanol und anderen niederen Alkoholen" wobei die Menge des Lösungsmittels der 24- bis 48-fachen Menge des Ausgangsmaterials entsprechen sollte;
b. Kühlen und Filtrieren des in Schritt a hergestellten Extrakts;
Leiten des Filtrats über eine mit Absorptionsharz gefüllte Säule;
Verwerfen der ausströmenden Flüssigkeit und Waschen mit Wasser, bis die ausströmende Flüssigkeit farblos wird; und
Verwerfen des Spülwassers;
c. Eluieren der mit Absorptionsharz gefüllten Säule, die in Schritt b mit Wasser gewaschen wird, mit einem oder mehreren Lösungsmitteln, gewählt aus der Gruppe, bestehend aus Ethanol, Methanol, Aceton, 50- bis 90%igem Ethanol, 30- bis 80%igem wässrigem Methanol und 60- bis 95%igem wässrigem Aceton;
Auffangen und Kondensieren der ausströmenden Flüssigkeit;
d. Zugeben von 60- bis 95%igem Alkohol zu der in Schritt c erhaltenen konzentrierten Lösung für eine Alkoholfällung;
Filtrieren und Gewinnen des Filtrats;
Kondensieren und Trocknen des Filtrats, um das Produkt zu erhalten.

2. Pharmazeutische Zusammensetzung nach Anspruch 1, umfassend:
12 bis 18 Gewichtsteile Pseudoprotodioscin (I),
1 Gewichtsteil Pseudoprotogracillin (II), und
1,2 bis 2,5 Gewichtsteile Dioscin (III).

3. Pharmazeutische Zusammensetzung nach Anspruch 1, wobei der Gesamtgehalt an steroidalen Saponinen in dem Extrakt nicht weniger als 65% (Gew./Gew.), berechnet bezogen auf Dioscin, beträgt.

4. Pharmazeutische Zusammensetzung nach Anspruch 1 oder 2, wobei der Gesamtgehalt der drei Arten an steroidalen Saponinen, Pseudoprotodioscin (I), Pseudoprotogracillin (II) und Dioscin (III), nicht weniger als 50% (Gew./Gew.) des Gehalts an steroidalen Saponinen insgesamt beträgt.

5. Pharmazeutische Zusammensetzung nach Anspruch 1, 3 oder 4, wobei der Extrakt den HPLC-Fingerabdruck aufweiset, wie er aus Fig. 1 ersichtlich ist, und wobei die charakteristischen Peaks des HPLC-Fingerabdrucks wie folgt sind:
Retentionszeit für Pseudoprotodioscin (I): 28,27 min;
Retentionszeit für Pseudoprotogracillin (II): 29,5 min; und
Retentionszeit für Dioscin (III): 57,10 min;
wobei die Chromatographiebedingungen für die HPLC wie folgt sind:
Chromatographiesäule: Alltima C18, 4,6 x 250 mm, 5 µm;
Gradientenelution;
Nachweis mittels eines Verdampfungs-Lichtstreu-Detektors;
Temperatur Driftröhre: 100°C; und
Gasdurchflussrate: 2,0 1/min.

6. Pharmazeutische Zusammensetzung nach Anspruch 1, wobei das Verfahren in Schritt b weiterhin einen Schritt der Kondensation unter Dekompression vor dem Kühlen und Filtrieren umfasst, wenn der Extrakt Methanol, Ethanol, n-Butanol oder andere niedere Alkohole enthält.

7. Pharmazeutische Zusammensetzung nach einem der Ansprüche 1 bis 6, welche die steroidalen Saponine oder die Extrakte von *Dioscorea panthaica* Prain et Burkill und *Dioscorea nipponica* Makino als Wirkstoff und pharmazeutisch annehmbare Adjuvanzien umfasst.

8. Pharmazeutische Zusammensetzung nach Anspruch 7, wobei die pharmazeutische Zusammensetzung in Form einer Tablette, einer Kapsel, einer Weichkapsel, eines Granulats, einer Flüssigkeit zur oralen Anwendung, eines Tropfenpräparats, einer Pille oder eines Injektionspräparats vorliegt.

9. Verfahren zur Herstellung der pharmazeutischen Zusammensetzung nach einem der Ansprüche 1 bis 8, welches die folgenden Schritte umfasst:
Abwiegen von Furostanol-Saponinen der allgemeinen Formel A und von Spirostanol-Saponin der allgemeinen Formel C;
Mischen der Saponine in einem Gewichtsverhältnis von 5 bis 20 Teilen Furostanol-Saponin, wobei das Furostanol-Saponin Pseudoprotodioscin (I) ist, 1 bis 3 Teilen Furostanol-Saponin, wobei das Furostanol-Saponin Pseudoprotogracillin (II) ist, und 1 bis 5 Teilen Spirostanol-Saponin oder Sapogenin, wobei das Spirostanol-Saponin oder Sapogenin Dioscin (III) ist; und
Hinzufügen von pharmazeutisch annehmbaren Adjuvanzien, um die pharmazeutische Zusammensetzung zu erhalten.

10. Verfahren zur Herstellung der pharmazeutischen Zusammensetzung nach einem der Ansprüche 1 bis 8, welches die folgenden Schritte umfasst:
a. Gewinnen der Wurzelstöcke der Kräuter *Dioscorea panthaica* Prain et Burkill und *Dioscorea nipponica* Makino oder der Wurzelstöcke von frisch gesammelten Pflanzen von *Dioscorea panthaica* Prain et Burkill und *Dioscorea nipponica* Makino als Ausgangsmaterial; Zerteilen oder Zerdrücken der Wurzelstöcke; Extrahieren mit einem oder mehreren Lösungsmitteln, gewählt aus der Gruppe, bestehend aus Wasser, Methanol, Ethanol, n-Butanol und anderen niederen Alkoholen, wobei die Menge des Lösungsmittels der 24- bis 48-fachen Menge des Ausgangsmaterials entsprechen sollte;
b. Kühlen und Filtrieren des in Schritt a hergestellten Extrakts; Leiten des Filtrats über eine mit Absorptionsharz gefüllte Säule; Verwerfen der ausströmenden Flüssigkeit und Waschen mit Wasser, bis die ausströmende Flüssigkeit farblos wird; und Verwerfen des Spülwassers;
c. Eluieren der mit Absorptionsharz gefüllte Säule, die in Schritt b mit Wasser gewaschen wird, mit einem oder mehreren Lösungsmitteln, gewählt aus der Gruppe, bestehend aus Ethanol, Methanol, Aceton, 50- bis 90%igem Ethanol, 30- bis 80%igem wässrigem Methanol und 60- bis 95%igem wässrigem Aceton; Auffangen und Kondensieren der ausströmenden Flüssigkeit;
d. Zugeben von 60- bis 95%igem Alkohol zu der in Schritt c erhaltenen konzentrierten Lösung für eine Alkoholfällung; Gewinnen des Filtrats nach der Filtration;
e. Kondensieren und Trocknen des in Schritt d erhaltenen Filtrats; Einzufügen von pharmazeutisch annehmbaren Adjuvanzien, um die pharmazeutische Zusammensetzung zu erhalten.

11. Verfahren nach Anspruch 10, wobei das Verfahren in Schritt b weiterhin einen Schritt der Kondensation unter Dekompression vor dem Kühlen und Filtrieren umfasst, wenn der Extrakt Methanol, Ethanol, n-Butanol oder andere niedere Alkohole enthält.

12. Verwendung der pharmazeutischen Zusammensetzung nach Anspruch 1 bis 8 bei der Herstellung eines Arzneimittels zur Behandlung und Prävention von cerebrokardialen Gefäßerkrankungen.

13. Verwendung nach Anspruch 12, wobei die Erkrankungen koronare Herzerkrankungen, Herzschmerzen, Herzinfarkt, Herzrhythmusstörungen, Hyperlipämie oder ischämische cerebrovaskuläre Erkrankungen einschließen.

## Revendications

1. Composition pharmaceutique contenant des saponines stéroïdes, comprenant :
5 à 20 parties en poids de saponine de type furostanol, ladite saponine de type furostanol étant la pseudoprotodioscine (I) et étant représentée par la formule générale A dans laquelle R₁ = 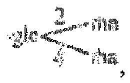
1 à 3 parties en poids de saponine de type furostanol, ladite saponine de type furostanol étant la pseudoprotogracilline (II) et étant représentée par la formule générale dans laquelle R₁ = 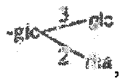
1 à 5 parties en poids de saponine de type spirostanol ou sapogénine, ladite saponine de type spirostanol ou sapogénine étant la dioscine (III) et étant représentée par la formule générale C dans laquelle R₃ = 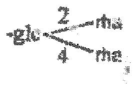 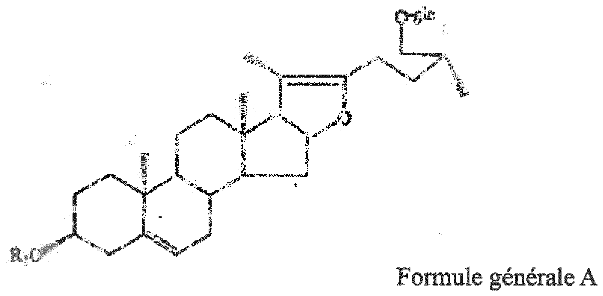 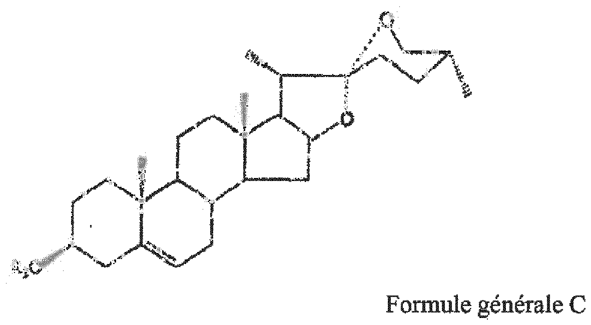 lesdites saponines astéroïdes provenant d'extraits de *Dioscorea panthaica* Prain et Burkill et *Dioscorea nipponica* Makino, étant toutes deux des espèces de Dioscorea, Dioscoreaceae, et
ledit extrait étant préparé par la méthode comprenant les étapes consistant à :
a. prendre des rhizomes de *Dioscorea panthaica* Prain et Burkill et *Dioscorea nipponica* Makino, ou les rhizomes fraîchement collectés de *Dioscorea panthaica* Prain et Burkill et *Dioscorea nipponica* Makino comme matière première ;
écraser et de couper les rhizomes ;
extraire avec un ou plus d'un types de solvants choisis parmi un groupe constitué de l'eau, du méthanol, de l'éthanol, du n-butanol et d'autres alcools gras inférieurs, la quantité dudit solvant étant 24 à 48 fois supérieure à celle de matière première ;
b. laisser refroidir et filtrer l'extrait préparé à l'étape a ;
faire migrer le filtrat à travers une colonne de résine absorbante,
éliminer l'effluent et laver à l'eau jusqu'à ce que l'effluent soit incolore ;
éliminer l'eau de rinçage ;
c. éluer la colonne de résine absorbante qui est lavée à l'eau à l'étape b avec un ou plus d'un types de solvants choisis dans un groupe constitué de l'éthanol, du méthanol, de l'acétone, d'éthanol 50 - 90 %, de méthanol aqueux 30 - 80 % et d'acétone aqueux 60 - 95 % ;
récolter et condenser l'effluent ;
d. ajouter 60 à 95 % d'alcool à la solution concentrée obtenue à l'étape c pour la précipitation à l'alcool, filtrer et récolter le filtrat ;
condenser et sécher le filtrat pour obtenir un produit.

2. Composition pharmaceutique selon la revendication 1 laquelle comprend :
12 à 18 parties en poids de pseudoprotodioscine (I),
1 partie en poids de pseudoprotogracilline (II) et 1,2 à 2,5 parties en poids de dioscine (III).

3. Composition pharmaceutique selon la revendication 1, dans laquelle dans ledit extrait, le contenu de saponine stéroïde totale n'est pas inférieur à 65 % (poids/poids) rapporté à la dioscine.

4. Composition pharmaceutique selon la revendication 1 ou 2, dans laquelle dans ledit extrait, le contenu total des trois types de saponines stéroïdes, pseudoprotodioscine (I), pseudoprotogracilline (II) et dioscine (III), n'est pas inférieur à 50 % (poids/poids) du contenu de saponines stéroïdes totales.

5. Composition pharmaceutique selon la revendication 1, 3 ou 4, dans laquelle ledit extrait présente une empreinte HPLC telle que montrée à la figure 1, dans laquelle les pics caractéristiques de ladite empreinte HPLC sont comme suit :
temps de rétention pour la pseudoprotodioscine (I) : 28,27 min ;
temps de rétention pour la pseudoprotogracilline (II) : 29,5 main ; et
temps de rétention pour la dioscin, (III) : 57,10 min ;
les conditions chromatographiques de HPLC sont les suivantes :
colonne chromatographique : Alltima C18 4,6 x 250 mm, 5 µm ;
gradient d'élution ;
déterminé avec un détecteur de diffusion de la lumière après évaporation ;
température du tube de dérive : 100°C ; et
débit de gaz : 2,0 1/min.

6. Composition pharmaceutique selon la revendication 1, dans la elle l'étape b comprend en outre une étape de décompression et condensation avant refroidissement et filtration lorsque l'extrait contient du méthanol, de l'éthanol, du n-butanol ou d'autres alcools gras inférieurs.

7. Composition pharmaceutique selon les revendications 1 à 6, laquelle comprend lesdites saponines stéroïdes ou les extraits de *Dioscorea panthaica* Prain et Burkill et *Dioscorea nipponica* Makino en tant que principe actif et des adjuvants pharmaceutiquement acceptables.

8. Composition pharmaceutique selon la revendication 7, dans la elle ladite composition pharmaceutique est sous la forme de comprimé, gélule, gélule à coque souple, granulés, solution buvable, dripping pill ou injection.

9. Méthode de préparation de la composition pharmaceutique selon l'une quelconque des revendications 1 à 8, qui comprend les étapes suivantes :
prélever par pesée des saponines de type furostanol de formule générale A et une saponine de type spirostanol de formule générale C, les mélanger dans le rapport pondéral de 5 à 20 parties de saponine de type furostanol, ladite saponine de type furostanol étant la pseudoprotodioscine (I), de 1 à 3 parties en poids de saponine de type furostanol, ladite saponine de type furostanol étant la pseudoprotogracilline (II), et de 1 à 5 parties de saponine de type spirostanol ou sapogénine, ladite saponine de type spirostanol ou sapogénine étant la dioscine (III) ; ajouter des adjuvants pharmaceutiquement acceptables pour obtenir la composition pharmaceutique.

10. Méthode de préparation de la composition pharmaceutique selon l'une quelconque des revendications 1 à 8, qui comprend les étapes suivantes :
a. prendre des rhizomes de *Dioscorea panthaica* Prain et Burkill et *Dioscorea nipponica* Makino, ou des rhizomes fraîchement collectés de *Dioscorea panthaica* Pralin et Burkill et *Dioscorea nipponica* Makino comme matière première, couper ou écraser les rhizomes, extraire avec un ou plus d'un types de solvants choisis parmi un groupe constitué de l'eau, du méthanol, de l'éthanol, du n-butanol et d'autres alcools gras inférieurs, la quantité dudit solvant étant 24 à 48 fois supérieure à celle de matière première ;
b, laisser refroidir et filtrer l'extrait préparé à l'étape a ; faire migrer le filtrat à travers une colonne de résine absorbante ; éliminer l'effluent et laver à l'eau jusqu'à ce que l'effluent soit incolore ; éliminer l'eau de rinçage ;
c. éluer la colonne de résine absorbante qui est lavée à l'eau à l'étape b avec un ou plus d'un types de solvants choisis dans un groupe constitué de l'éthanol, du méthanol, de l'acétone, d'éthanol 50 - 90 %, de méthanol aqueux 30 - 80 % et d'acétone aqueux 60 - 95 % ; récolter et condenser l'effluent ;
d. ajouter 60 à 95 % d'alcool à la solution concentrée obtenue à l'étape c pour la précipitation à l'alcool, récolter le filtrat après filtration ;
e. condenser et sécher le filtrat de l'étape d, ajouter des adjuvants pharmaceutiquement acceptables pour obtenir la composition pharmaceutique.

11. Méthode selon la revendication 10, dans laquelle à l'étape b, elle comprend en outre une étape de décompression et condensation avant le refroidissement et la filtration lorsque l'extrait contient du méthanol, de l'éthanol, du n-butanol ou d'autres alcools gras inférieurs.

12. Utilisation de la composition pharmaceutique des revendications 1 à 8, dans la préparation de la composition pharmaceutique pour le traitement et la prévention de maladies cérébro-cardiovasculaires.

13. Utilisation selon la revendication 12, dans laquelle lesdites maladies sont une coronaropathie, l'angor, l'infarctus du myocarde, l'arythmie, l'hyperlipémie ou des maladies cérébro-vasculaires ischémiques.
